(19) Europäisches Patentamt
European Patent Office
Office européen des brevets

(11) EP 2 351 553 A1

(12) **EUROPEAN PATENT APPLICATION**
published in accordance with Art. 153(4) EPC

(43) Date of publication:
**03.08.2011 Bulletin 2011/31**

(21) Application number: **09825917.9**

(22) Date of filing: **12.11.2009**

(51) Int Cl.:
***A61K 8/73*** (2006.01) ***A61K 8/46*** (2006.01)
***A61Q 5/02*** (2006.01) ***A61Q 19/02*** (2006.01)

(86) International application number:
**PCT/JP2009/006049**

(87) International publication number:
**WO 2010/055664 (20.05.2010 Gazette 2010/20)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **12.11.2008 JP 2008289893**

(71) Applicant: **Lion Corporation**
**Tokyo 130-8644 (JP)**

(72) Inventors:
- **KOHNO, Youichirou**
  **Tokyo 130-8644 (JP)**
- **SATO, Nobuyasu**
  **Tokyo 130-8644 (JP)**
- **OSAKO, Yoko**
  **Tokyo 130-8644 (JP)**
- **TANABE, Miho**
  **Tokyo 130-8644 (JP)**
- **YAMAGATA, Yoshifumi**
  **Tokyo 130-8644 (JP)**

(74) Representative: **Denjean, Eric**
**Cabinet Laurent & Charras**
**"Le Contemporain"**
**50, Chemin de la Bruyère**
**69574 Dardilly Cedex (FR)**

# (54) CLEANSER COMPOSITION

(57) There is provided a detergent composition that exhibits excellent conditioning performance,
the hair detergent composition including an anionic surfactant and a silane-modified cationized cellulose, wherein the anionic surfactant is preferably a polyoxyethylene alkyl ether sulfate.

Also provided is a skin detergent composition including an anionic surfactant and a silane-modified cationized cellulose.

EP 2 351 553 A1

## Description

### TECHNICAL FIELD

The present invention relates to a detergent composition.
Priority is claimed on Japanese Patent Application No. 2008-289893, filed November 12, 2008, the content of which is incorporated herein by reference.

### BACKGROUND ART

Conventionally, in the hair detergent compositions such as shampoos and the skin detergent compositions such as body soaps, in addition to the surfactant which serves as a cleaning component, a conditioning agent has been added in order to improve the conditioning performance (such as the ease of finger combing during rinsing, stiffness during drying, and smooth feeling).
Cationized celluloses are available as compounds used as the conditioning agents (for example, refer to Patent Document 1). Since the cationized celluloses are generally dissolved and used in water or in a mixed solvent containing water (hereafter, sometimes referred to as an aqueous solvent) for such purposes, they are usually made into a particulate form and used in consideration of solubility. However, since the cationized celluloses exhibit a high level of hydrophilicity, water dispersibility is poor when they are made into a particulate form for use.
With respect to such a problem, it has been proposed to use a cationized cellulose with enhanced hydrophobicity and improved water dispersibility due to a glyoxal treatment (for example, refer to Patent Document 2). When a glyoxal treatment is conducted, glyoxal and a cationized cellulose form a cross-linkage through hemiacetal bonding, thereby enhancing the hydrophobicity. This cross-linkage is hydrolyzed due to alkali or heat. As a result, the cationized cellulose which has been subjected to a glyoxal treatment disperses due to excellent water dispersibility when introduced into water or aqueous solvent, and subsequently manifests excellent solubility due to alkali or heat.
However, although the cationized cellulose mentioned above which has been subjected to a glyoxal treatment exhibits excellent water dispersibility, the hair detergent composition to which the aforementioned cationized cellulose has been added generates a sense of friction between the hair and the finger during rinsing, and thus it cannot be said that the conditioning performance is satisfactory.
Note that with respect to the glyoxal treatment, alternative techniques have been studied since glyoxal has been designated as a mutagenic substance, and, for example, silane modification methods which use a silane compound have been proposed. As the silane modification methods, for example, there have been proposals of methods which use aminosilane or epoxysilane (Patent Documents 3 and 4), methods which use alkyltrialkoxyl silane, alkyltetraacyloxy silane, tetraalkoxy silane, and tetraacyloxy silane (Patent Documents 5 to 8), and the like.
In Patent Document 9, a silane-modified cationized cellulose obtained by treating a cationized cellulose with an aminosilane coupling agent has been disclosed.

### PRIOR ART DOCUMENTS

### PATENT DOCUMENTS

[Patent Document 1] Japanese Unexamined Patent Application, First Publication No. Sho 55-38813
[Patent Document 2] Japanese Laid-Open Patent Application No. 2000-319139
[Patent Document 3] Japanese Examined Patent Application, Second Publication No. Sho 51-2103
[Patent Document 4] Japanese Unexamined Patent Application, First Publication No. Sho 47-35073
[Patent Document 5] Japanese Examined Patent Application, Second Publication No. Hei 6-39481
[Patent Document 6] Japanese Unexamined Patent Application, First Publication No. Hei 8-183801
[Patent Document 7] Japanese Laid-Open Patent Application No. 2004-155805
[Patent Document 8] Japanese Unexamined Patent Application, First Publication No. Sho 61-195138
[Patent Document 9] Japanese Laid-Open Patent Application No. 2007-211167

### DISCLOSURE OF INVENTION

### PROBLEMS TO BE SOLVED BY THE INVENTION

The present invention has been developed in light of the above circumstances, and its object is to provide a

detergent composition that exhibits excellent conditioning performance.

MEANS TO SOLVE THE PROBLEMS

As a result of intensive and extensive investigation, the inventors of the present invention have discovered that excellent conditioning performance can be achieved by combining a specific surfactant with a specific cationized cellulose to complete the present invention.
That is, a hair detergent composition of the present invention which solves the above-mentioned problem is characterized by including an anionic surfactant and a silane-modified cationized cellulose.
In addition, a skin detergent composition of the present invention is characterized by including an anionic surfactant and a silane-modified cationized cellulose.

EFFECT OF THE INVENTION

According to the present invention, it is possible to provide a detergent composition that exhibits excellent conditioning performance.

EMBODIMENTS FOR CARRYING OUT THE INVENTION

A detergent composition of the present invention includes an anionic surfactant (hereafter, frequently referred to as a "component (A)") and a silane-modified cationized cellulose (hereafter, frequently referred to as a "component (B)").
Examples of the component (A) include sulfuric acid ester salt-based anionic surfactants, sulfonic acid salt-based anionic surfactants, carboxylic acid salt-based anionic surfactants, phosphoric acid ester salt-based anionic surfactants and the like. Of these, sulfuric acid ester salt-based anionic surfactants are preferred in terms of excellent stability when mixed with other components and also from the viewpoints of foaming properties and cost.
Examples of the salt include alkali metal salts such as sodium salts and potassium salts; alkaline earth metal salts such as calcium salts and magnesium salts; alkanolamine salts such as triethanolamines; or ammonium salts, and alkali metal salts are preferred.
Examples of the sulfuric acid ester salt-based anionic surfactants include alkyl sulfates, alkenyl sulfates, polyoxyalkylene alkyl ether sulfates, polyoxyalkylene alkenyl ether sulfates, polyoxyalkylene alkyl phenyl ether sulfates, polyoxyalkylene alkenyl phenyl ether sulfates, alkyl polyhydric alcohol ether sulfates, and the like.
More specifically, examples of the alkyl sulfates and the alkenyl sulfates include alkyl sulfates or alkenyl sulfates which have 10 to 20 carbon atoms.
Polyoxyalkylene alkyl ether sulfates, polyoxyalkylene alkenyl ether sulfates, polyoxyalkylene alkyl phenyl ether sulfates and polyoxyalkylene alkenyl phenyl ether sulfates are those which are formed by adding alkylene oxides to alkyl ether sulfates, alkenyl ether sulfates, alkyl phenyl ether sulfates and alkenyl phenyl ether sulfates, respectively.
As the alkylene oxide, either a single alkylene oxide of 2 to 4 carbon atoms or a mixture of ethylene oxide (EO) and propylene oxide (PO) is preferred. In the case of using a mixture of EO and PO, the ratio thereof in terms of molar ratio is preferably within a range so that EO/PO = 0.1/9.9 to 9.9/0.1. As the alkylene oxide, EO is particularly desirable.
As for the number of moles of alkylene oxide added, an average of 0.5 to 10 moles are preferred in the cases of polyoxyalkylene alkyl ether sulfates or polyoxyalkylene alkenyl ether sulfates. In addition, an average of 3 to 30 moles is preferred in the cases of polyoxyalkylene alkyl phenyl ether sulfates and polyoxyalkylene alkenyl phenyl ether sulfates.
In polyoxyalkylene alkyl ether sulfates, polyoxyalkylene alkenyl ether sulfates, polyoxyalkylene alkyl phenyl ether sulfates and polyoxyalkylene alkenyl phenyl ether sulfates, the alkyl groups or the alkenyl groups preferably have 8 to 20 carbon atoms. The alkyl groups or alkenyl groups may be linear or branched.
Examples of the alkyl polyhydric alcohol ether sulfates include alkyl glyceryl ether sulfates of 10 to 20 carbon atoms and the like.
As a sulfuric acid ester salt-based anionic surfactant, polyoxyalkylene alkyl ether sulfates are preferred in terms of excellent stability when mixed with other components and also from the viewpoints of foaming properties and cost.
Preferred examples ofpolyoxyethylene alkyl ether sulfates include compounds represented by general formula (1) shown below.
[Chemical Formula 1]

$$R^1O-(CH_2CH_2O)_{n_1}-SO_3M \quad \cdots (1)$$

[In formula (1), $R^1$ represents an alkyl group of 8 to 20 carbon atoms; $n_1$ represents the average number of moles of ethylene oxide added; M represents an alkali metal atom, an alkaline earth metal atom, an alkanolamine or ammonium.]

In the aforementioned general formula (1), $R^1$ represents an alkyl group of 8 to 20 carbon atoms. The number of carbon atoms of the alkyl group is preferably within a range from 8 to 18, more preferably from 10 to 16, and still more preferably from 12 to 13. When the number of carbon atoms in the $R^1$ group is equal to or greater than 8, the detergency improves since the hydrophobicity is enhanced. On the other hand, if the number of carbon atoms in the $R^1$ group is equal to or less than 20, the precipitation during storage can be suppressed since the solubility of the compound per se becomes favorable. The alkyl groups may be linear or branched.

$n_1$ represents the average number of moles of ethylene oxide added, and is preferably from 1 to 6, more preferably from 1 to 4, and still more preferably from 1 to 3.

M represents an alkali metal atom, an alkaline earth metal atom, an alkanolamine or ammonium.

Examples of the alkali metal atoms include sodium, potassium and the like.

Examples of the alkaline earth metal atoms include calcium, magnesium and the like.

Examples of the alkanolamines include triethanolamine and the like.

Among the above examples, as M, an alkali metal atom is preferred, and sodium is particularly preferred.

Examples of the sulfonic acid salt-based anionic surfactants include alkylbenzene sulfonates, alkane sulfonates, α-olefin sulfonates, α-sulfofatty acid salts, α-sulfofatty acid alkyl ester salts and the like.

More specifically, examples of the alkylbenzene sulfonates include linear or branched alkylbenzene sulfonates having an alkyl group of 8 to 18 carbon atoms.

Alkane sulfonates are also known as paraffin sulfonates and examples thereof include alkane sulfonates of 10 to 21 carbon atoms. As an alkane sulfonate, it is preferable to include a secondary alkane sulfonate. Preferred examples of alkane sulfonates include a mixture of secondary alkane sulfonates having 10 to 21 carbon atoms per molecule (preferably at least 80% by weight and more preferably at least 90% by weight of the secondary alkane sulfonates have 10 to 14 carbon atoms per molecule) and a small amount of primary alkane sulfonates, disulfonates or polysulfonates.

Examples of the α-olefin sulfonates include α-olefin sulfonates of 10 to 20 carbon atoms.

Examples of the α-sulfofatty acid salts include saturated or unsaturated α-sulfofatty acid salts of 8 to 20 carbon atoms.

Examples of the α-sulfofatty acid alkyl ester salts include a methyl, ethyl or propyl ester salt of the aforementioned α-sulfofatty acid salts.

Examples of the carboxylic acid salt-based anionic surfactants include polyoxyalkylene alkyl ether carboxylates, polyoxyalkylene alkenyl ether carboxylates, higher fatty acid salts (soap) of 10 to 20 carbon atoms and the like. Specific examples thereof include alkaline salts (such as sodium salts, potassium salts, calcium salts, magnesium salts, ammonium salts, triethanolamine salts and tripropanolamine salts) of fatty acids (such as lauric acid, myristic acid, palmitic acid, stearic acid, isostearic acid and oleic acid) and the like. Specific examples of preferred fatty acid alkaline salts include a single or mixed salt composed of sodium salts and potassium salts of lauric acid, myristic acid, palmitic acid, stearic acid, oleic acid, isostearic acid and the like. These salts may be used alone, or two or more types thereof may be mixed for use.

Examples of the alkylene oxide in polyoxyalkylene alkyl ether carboxylates and polyoxyalkylene alkenyl ether carboxylates include the same as those listed above as examples in the description for the aforementioned sulfuric acid ester salt-based anionic surfactants.

In polyoxyalkylene alkyl ether carboxylates and polyoxyalkylene alkenyl ether carboxylates, the alkyl groups or the alkenyl groups preferably have 10 to 20 carbon atoms. The alkyl groups or alkenyl groups may be linear or branched.

Examples of the phosphoric acid ester based anionic surfactants include long-chain monoalkyl phosphates, long-chain dialkyl phosphates, long-chain sesquialkyl phosphates, polyoxyethylene monoalkyl phosphates, polyoxyethylene dialkyl phosphates, polyoxyethylene sesquialkyl phosphates and the like. The "long chain" alkyl group represents an alkyl group having 8 or more carbon atoms, and preferably 10 to 20 carbon atoms.

As the component (A), either commercially available products may be used or products synthesized by a known method may be used.

For example, the polyoxyethylene alkyl ether sulfate represented by the aforementioned general formula (1) can be produced in the following manner.
A polyoxyethylene alkyl ether (alcohol ethoxylate) can be obtained by adding ethylene oxide to a higher alcohol ($R^1$-OH). The aforementioned polyoxyethylene alkyl ether is sulfonated or sulfated with Sulfan. As a result, a polyoxyethylene alkyl ether sulfate can be obtained.
Either a single type of component (A) may be used alone, or two or more types thereof may be mixed for use. The content of the component (A) within the detergent composition, relative to the total weight of the detergent composition, is preferably from 1 to 20% by weight, more preferably from 3 to 18% by weight, and still more preferably from 5 to 15% by weight. When the content is at least as large as the lower limit of the aforementioned range, detergency or the like is improved, whereas if the content is equal to or less than the upper limit of the aforementioned range, the level of irritation during use can be reduced.
Examples of the component (B) (silane-modified cationized cellulose) include the cationized celluloses which have been treated with an aminosilane coupling agent.
Although the aforementioned component (B) can be produced, for example, by the production method described in Japanese Laid-Open Patent Application No. 2007-216089, in the present invention, the component (B) is preferably produced by a production method that includes the following steps (1) to (6).
Step (1): A step of cationizing a water-soluble cellulose ether in a mixed solvent composed of water-compatible organic solvent and water under the presence of an alkali, thereby obtaining a slurry that contains a cationized cellulose.
Step (2): A step of adding an acid to the slurry obtained in the aforementioned step (1) to neutralize the alkali.
Step (3): A step of adding a water-compatible organic solvent or a mixed solvent composed of a water-compatible organic solvent and water so that the water content in the entire solvent that comes into contact with the cationized cellulose becomes 10% by weight or less, with respect to the cationized cellulose following neutralization.
Step (4): A step of reacting the cationized cellulose obtained in the aforementioned step (3) with an aminosilane compound.
Step (5): A step of adding an aminosilane compound to the slurry obtained in the aforementioned step (1) or to the cake thereof, thereby treating the aforementioned cationized cellulose with the aforementioned aminosilane compound, the step in which the amount of the aforementioned aminosilane compound added is from 0.05 to 20% by weight relative to the aforementioned water-soluble cellulose ether, and the treatment of the aforementioned cationized cellulose with the aforementioned aminosilane compound is conducted by the reaction under alkaline conditions at a pH of 10 or more.
Step (6): A step of conducting a first drying treatment in which the product obtained in the aforementioned step (5) is processed at a temperature of 50 to 120°C and a degree of vacuum of 13.4 to 53.3 kPa, and a second drying treatment in which the aforementioned product following the first drying treatment is processed at a temperature of 90 to 150°C and a degree of vacuum of 13.3 kPa or less.
Step (7): A step of adding an acid to the reaction product obtained in the aforementioned step (5) for neutralization.
Step (8): A step of conducting a first drying treatment in which the product obtained in the aforementioned step (7) is processed at a temperature of 50 to 120°C and a degree of vacuum of 13.4 to 53.3 kPa, and a second drying treatment in which the aforementioned product following the first drying treatment is processed at a temperature of 90 to 150°C and a degree of vacuum of 13.3 kPa or less.
Step (9): A step of conducting a first drying treatment in which the product obtained in the aforementioned step (3) is processed at a temperature of 50 to 120°C and a degree of vacuum of 13.4 to 53.3 kPa, and a second drying treatment in which the aforementioned product following the first drying treatment is processed at a temperature of 90 to 150°C and a degree of vacuum of 13.3 kPa or less.
The component (B) produced by such a production method exhibits excellent water dispersibility and disperses readily within a short period of time when introduced into aqueous solvents such as water and a mixed solvent composed of water and a water-compatible organic solvent. The component (B) also exhibits excellent solubility in aqueous solvents, and is also superior in terms of safety when compared with the conventional cationized cellulose which has undergone a glyoxal treatment.
A more detailed description of each of the steps is provided below.

<Step (1)>

In step (1), a water-soluble cellulose ether is cationized in a mixed solvent composed of water-compatible organic solvent and water under the presence of an alkali, thereby obtaining a slurry that contains a cationized cellulose. Examples of the water-soluble cellulose ethers include hydroxyalkyl cellulose ether. Hydroxyalkyl cellulose ether is a substance in which hydroxyakyl groups are bonded as substituents to the hydroxyl groups of cellulose.
The aforementioned hydroxyalkyl groups are groups represented by the general formula $-(A-O)_nH$. In the formula, A represents alkylene groups of 2 to 3 carbon atoms, of which ethylene groups or propylene groups are preferable, and ethylene groups are more preferable. n represents the average number of moles of alkylene oxide added. With respect to the aforementioned average number of moles added, it is preferable to have 0.5 to 3.5 moles relative to 1 mole of

glucose residue (unit skeleton) of water-soluble cellulose ether, and 1 to 2.5 moles are more preferable.
Hydroxyalkyl cellulose ether may also have substituents other than hydroxyalkyl groups. Examples of the aforementioned substituents include alkyl groups of 1 to 3 carbon atoms and the like.
Specific examples of the hydroxyalkyl cellulose ethers include hydroxyethyl cellulose (HEC), hydroxypropyl cellulose (HPC), methyl-hydroxyethyl cellulose (MHEC), methyl-hydroxypropyl cellulose (MHPC), ethyl-hydroxyethyl cellulose (EHEC) and the like. Among these, HEC is preferable due to its low cost and its common use in applications involving thickeners and the like. Any one of these may be used alone, or two or more types thereof may be used in combination.
With respect to the hydroxyalkyl cellulose ether, a commercially available product may be used, or it may be synthesized. For example, hydroxyalkyl cellulose ether may be synthesized by subjecting cellulose to an alkali treatment to obtain alkali cellulose, and then by reacting this with alkylene oxide.
Examples of commercially available products include HEC AL-15, AH-15, AX-15, SW-25F, SG-25F, and SY-25F manufactured by Sumitomo Seika Chemicals Co., Ltd., as well as HEC Daicel SE550, SE600, and SE900 manufactured by Daicel Chemical Industries, Ltd.
As for the viscosity of water-soluble cellulose ether, it is preferable that viscosity in an aqueous solution of 2% by weight at 20°C be 5 to 35,000 mPa·s. Viscosity refers to viscosity after one minute from the start of measurement by a Brookfield viscometer.

As a water-compatible organic solvent, it is sufficient if the substance forms a uniform solution when mixed with water, and examples thereof include alcohols of 1 to 4 carbon atoms, acetone and the like. Among these, alcohols of 1 to 4 carbon atoms are preferable. Specific examples thereof include methanol, ethanol, isopropanol, n-propanol, t-butyl alcohol and the like. Among these, ethanol, isopropanol, and t-butyl alcohol are preferable from the viewpoints of price and safety.
With respect to the proportion of water in the mixed solvent, from the viewpoints of inhibiting side reactions and efficiently promoting cationization reaction, 12 to 30% by weight is preferable, and 12 to 20% by weight is more preferable. By setting the proportion at least as large as the lower limit, the cationization reaction can be promoted more efficiently. Setting the proportion equal to or more than the upper limit is undesirable from the viewpoints of handling properties and manufacturability, as the cationized cellulose and water-soluble cellulose ether that are produced dissolve to lower the yield, and as gelation occurs due to the partial dissolution thereof in water.
As for the amount of mixed solvent to be used, from the viewpoints of avoiding localized progression of cationization of the water-soluble cellulose ether and enhancing volumetric efficiency of the reaction vessel, 200 to 1,500 parts by weight relative to 100 parts by weight of the water-soluble cellulose ether are preferable, and 300 to 800 parts by weight are more preferable.

Examples of alkali include alkali metal hydroxides such as sodium hydroxide, potassium hydroxide and lithium hydroxide. Among these, sodium hydroxide is preferable due to its low cost.
With respect to the amount of alkali to be used, an amount such that the alkali content becomes 0.1 to 10% by weight relative to the water-soluble cellulose ether is preferable.

Cationization of water-soluble cellulose ethers may be accomplished by reacting a water-soluble cellulose ether and a cationizing agent.
As a cationizing agent, it is sufficient if the substance reacts with hydrogen atoms (active hydrogen) of hydroxyl groups of water-soluble cellulose ether, and imparts cationic properties to the water-soluble cellulose ether, and specific examples thereof include glycidyl trialkyl ammonium halides such as glycidyl trimethyl ammonium chloride, glycidyl triethyl ammonium chloride, glycidyl trimethyl ammonium bromide, and glycidyl triethyl ammonium bromide; and ammonium halide compounds such as dimethyldiallyl ammonium chloride, methacryloyloxyethylene trimethyl ammonium chloride, and 3-chloro-2-hydroxypropyl trimethyl ammonium chloride. Among these, from the viewpoints of low cost and high reactivity, glycidyl trimethyl ammonium chloride is preferable.

With respect to the amount of cationizing agent to be used, from the viewpoint of raising the yield of cationized cellulose and from the viewpoint of avoiding loss of economic benefit due to effects that do not compensate for the amount used, it is preferable to have an amount that constitutes 0.1 to 1.4 moles relative to glucose residue unit skeleton in the water-soluble cellulose ether, and an amount that constitutes 0.3 to 1.2 moles is more preferable.

The reaction of water-soluble cellulose ether and cationizing agent can be conducted, for example, by mixing and stirring the water-soluble cellulose ether, the aforementioned mixed solvent and an alkali substance, after which the cationizing agent is added, and the resulting mixture is heated to a predetermined reaction temperature.
With respect to the reaction temperature in this case, from the viewpoint of accelerating reaction and shortening the reaction time, and from the viewpoint of preventing the reaction from proceeding too rapidly, the reaction temperature is usually within the range from 40 to 60°C, and preferably from 45 to 55°C.
Although the reaction time cannot be determined in general terms since it depends on the reaction temperature, it is usually about 2 to 4 hours.

In this manner, a slurry containing cationized cellulose can be obtained.
There are no particular limitations on the degree of cationization of the aforementioned cationized cellulose, and it may

be appropriately selected in accordance with the degree of cationization of the silane-modified cationized cellulose (component (B)) that is ultimately obtained. The degree of cationization is preferably from 0.3 to 2.5% by weight and more preferably from 0.5 to 2.0% by weight.
When the aforementioned degree of cationization is 0.3% by weight or higher, the cationic properties of the aforementioned cationized cellulose, and consequently the cationic properties of the silane-modified cationized cellulose that is ultimately obtained, are enhanced, and the functions thereof (such as thickening properties) are also improved. When the degree of cationization is 2.5% by weight or less, reactivity with aminosilane compounds is satisfactory, and the water dispersibility of the silane-modified cationized cellulose is also improved.
In addition, the degree of cationization of the silane-modified cationized cellulose is preferably from 0.5 to 2.7% by weight, and more preferably 0.7 to 2.2% by weight. When the aforementioned degree of cationization is 0.5% by weight or higher, excellent solubility and water dispersibility can be achieved. When the degree of cationization is 2.7% by weight or less, adequate levels of solubility and water dispersibility of the silane-modified cationized cellulose can also be achieved.
Here, the degree of cationization of cationized cellulose refers to a proportion of nitrogen atoms per unit skeleton of glucose residue of the aforementioned cationized cellulose. The degree of cationization may be measured by the method described on the page for O-[2-hydroxy-3-(trimethylammonio)propyl]hydroxyethyl cellulose chloride in Japanese Standards of Quasi-Drug Ingredients 2006 (Yakuji Nippo, Ltd.). The aforementioned nitrogen atoms originate from the cationizing agent, and the degree of cationization may be controlled by controlling the amount of cationizing agent to be used, or the like.

<Step (2)>

In the step (2), the alkali is neutralized by adding an acid to the slurry obtained in the aforementioned step (1). Examples of the acid include strong acids such as sulfuric acid, hydrochloric acid, and nitric acid, and weak acids such as acetic acid and phosphoric acid. Among these, hydrochloric acid, sulfuric acid and nitric acid are preferable due to their low cost.
With respect to the amount of acid to be used, it suffices if it is appropriately adjusted so that pH falls within the desired range described later when the final silane-modified cationized cellulose is prepared as an aqueous solution. Since the aminosilane compound used in the step (4) is alkaline, it is preferable that the amount be such that the pH of the slurry following addition of the aforementioned acid is lower than the aforementioned desired pH. More specifically, it is preferable that the amount be such that the pH of the slurry following addition of the aforementioned acid be 2.0 to 6.0, more preferably 3.5 to 5.5, under conditions of 25°C. When the aforementioned pH is within the aforementioned range, the water dispersibility of the silane-modified cationized cellulose that is ultimately obtained is satisfactory, and the solubility thereof in water is also favorable.

<Step (3)>

The water used during cationization remains in the slurry obtained in the aforementioned step (2) or in the step (7) to be described later, and water content in the entire solvent in the aforementioned slurry is usually 12 to 30% by weight. In the step (3), a water-compatible organic solvent or a mixed solvent composed of water and a water-compatible organic solvent is added to the cationized cellulose after the aforementioned neutralization so that water content in the entire solvent that is brought into contact with the pertinent cationized cellulose is 10% by weight or less.
Examples of the water-compatible organic solvent include the same water-compatible organic solvents described in the aforementioned step (1).
With respect to the proportion of water in the mixed solvent composed of water and a water-compatible organic solvent, it is acceptable if water content in the entire solvent (mother liquor) contained in the slurry of cationized cellulose following addition of the pertinent mixed solvent or in the cake obtained by subjecting the aforementioned slurry to a deliquoring treatment is within a range of 10% by weight or less, and it may be appropriately selected in accordance with the amount of water in the slurry or cake to which the pertinent mixed solvent is added.
Specific examples of the methods for carrying out the step (3)include the following methods (3-1), (3-2) and the like.
Method (3-1): A method in which a water-compatible organic solvent or a mixed solvent composed of a water-compatible organic solvent and water is added to the slurry obtained in the aforementioned step (2) or in the step (7) described later, followed by mixing and stirring.
Method (3-2): A method in which the slurry obtained in the aforementioned step (2) or in the step (7) described later is subjected to a deliquoring treatment, followed by addition of a water-compatible organic solvent or a mixed solvent composed of water-compatible organic solvent and water to the obtained cake.
More specific examples of the method (3-2) include the following methods (3-2a), (3-2b) and the like.

Method (3-2a): A method in which the neutralized slurry obtained in the aforementioned step (2) or in the step (7) described later is deliquored, followed by redispersing of the obtained cake in the water-compatible organic solvent or in the mixed solvent to produce a slurry.
Method (3-2b): A method in which the neutralized slurry obtained in the aforementioned step (2) or in the step (7) described later is deliquored, followed by showering of the water-compatible organic solvent or mixed solvent onto the obtained cake.
In those cases where the cake is treated by showering, a continuous treatment method may also be adopted in which the cake is placed on a belt conveyor or the like, and showering is conducted thereon.
In addition, after these treatments, a deliquoring treatment may be further conducted in order to remove the water-compatible organic solvent or mixed solvent which has been employed.
In the cases of method (3-1) and (3-2a), the aforementioned solvent may be added so that water content in the mother liquor contained in the slurry following addition of the water-compatible organic solvent or mixed solvent composed of water and water-compatible organic solvent is 10% by weight or less.
Further, in those cases where the cake is treated by showering as in the method (3-2b), showering may be conducted so that water content in the mother liquor contained in the cake is ultimately 10% by weight or less.
In the method (3-2), there are no particular limitations on the deliquoring treatment method, and conventionally known solid-liquid separation methods such as filtration and centrifugal separation can be employed. For example, it may be conducted by using a centrifugal deliquoring device which uses a filter cloth.
It is preferable that the deliquoring treatment be conducted so that solid content in the cake is from 40 to 70% by weight. The amount of the aforementioned solid content is calculated from the differential amount before and after 1 g of cake is dried for 2 hours at 105°C.

Note that water content in the aforementioned entire solvent of water-compatible organic solvent or mixed solvent can be confirmed by, for example, a method in which the slurry is left to stand or subjected to centrifugation, followed by extraction of the resulting supernatant to measure the water content, or a method in which the slurry or cake following addition of the water-compatible organic solvent or mixed solvent is subjected to a deliquoring treatment to measure the water content of the deliquored liquid.
The water content in liquid may be measured by the Karl Fischer method using a commercially available water-content measuring device such as the AQV-7 Trace Water Measuring Device manufactured by Hiranuma Sangyo Corporation.

Following the aforementioned neutralization, the cationized cellulose contains a salt that is produced by neutralization. Although the treatment by water-compatible organic solvent or mixed solvent in the present step may be combined with a purification step that cleans and removes this salt, when the water content in the water-compatible organic solvent or mixed solvent that is used as the cleaning fluid is low, efficiency for removing the neutralized salt declines, and there is a risk that the neutralized salt may remain in the obtained cationized cellulose.
For this reason, from the viewpoint of efficiently removing the neutralized salt, before addition of the water-compatible organic solvent or mixed solvent for adjusting the water content to 10% by weight or less, it is preferable to separately conduct cleaning (purification) of the cationized cellulose using a mixed solvent composed of water-soluble organic solvent and water with a water content of about 15 to 30% by weight.

<Step (4)>

In the step (4), the cationized cellulose obtained in the aforementioned step (3) is allowed to react with an aminosilane compound.
Examples of the aminosilane compound include 3-aminopropyltrimethoxy silane, 3-aminopropyltriethoxy silane, 3-aminopropylmethyldimethoxy silane, 3-aminopropyltrimethylethoxy silane, N-2-aminoethyl-3-aminopropyltrimethoxy silane, N-2-aminoethyl-3-aminopropyltriethoxy silane, 3-aminopropyldiethoxy silane, 4-aminobutylmethyldiethoxy silane and N-2-carboethoxyethyl-3-aminopropyltriethoxy silane.
Among these, 3-aminopropyltriethoxy silane, N-2-aminoethyl-3-aminopropyltriethoxy silane, 3-aminopropyltrimethylethoxy silane, 3-aminopropyldiethoxy silane, 4-aminobutylmethyldiethoxy silane and N-2-carboethoxyethyl-3-aminopropyltriethoxy silane are preferred from the viewpoint of preventing the release of methanol when the silane-modified cationized cellulose that is ultimately obtained is used in shampoo, body soap or the like. As the aminosilane compound, commercially available products such as KBE-903, KBE-603, and KBE-9103 manufactured by Shin-Etsu Chemical Co., Ltd., and AY43-059 manufactured by Dow Coming Toray Co., Ltd. can be used.
The amount of aminosilane compound to be used is preferably from 0.3 to 10% by weight, more preferably from 0.5 to 10% by weight, still more preferably from 0.9 to 5% by weight, and particularly preferably from 0.9 to 3% by weight, relative to the amount of water-soluble cellulose ether used as a raw material of the cationized cellulose that is reacted with the pertinent aminosilane compound. When the amount of the aforementioned aminosilane compound used is less than 0.5% by weight, the water dispersibility may be adversely affected. On the other hand, when the amount exceeds 10% by weight, although the water dispersibility remains favorable, the amount of active ingredient of cationized cellulose

reduces, and also the cost increases at the same time, which is undesirable from an industrial viewpoint.

<Step (5)>

In the step (5), an aminosilane compound is added to the slurry obtained in the aforementioned step (1) or to the cake thereof, thereby treating the aforementioned cationized cellulose with the aforementioned aminosilane compound.
The amount of aminosilane compound to be added is preferably from 0.05 to 20% by weight, more preferably from 0.1 to 15% by weight, and still more preferably from 0.5 to 15% by weight, relative to the aforementioned water-soluble cellulose ether.
In the step (5) a reaction is conducted under alkaline conditions, preferably at a pH of 10 or more, more preferably at a pH of 11 or more.
When the amount of the aforementioned aminosilane compound added and the pH are within the above-mentioned ranges, a favorable level of water dispersibility can be obtained.

<Step (6)>

In the step (6), a first drying treatment in which the product obtained in the aforementioned step (5) is processed at a temperature of 50 to 120°C and a degree of vacuum of 13.4 to 53.3 kPa, and a second drying treatment in which the aforementioned product following the first drying treatment is processed at a temperature of 90 to 150°C and a degree of vacuum of 13.3 kPa or less are conducted. Here, in the present description and the claims, the expression "degree of vacuum " describes a pressure based on the absolute pressure.
The temperature of the aforementioned first drying treatment is preferably from 50 to 120°C, more preferably from 60 to 110°C, and still more preferably from 70 to 100°C.
The degree of vacuum of the aforementioned first drying treatment is preferably from 13.4 to 53.3 kPa, more preferably from 13.4 to 46.6 kPa, and still more preferably from 13.4 to 39.9 kPa.
When the aforementioned temperature and the degree of vacuum are within the above-mentioned ranges, the solvent removal efficiency and the color tone will be satisfactory.
The temperature of the aforementioned second drying treatment is preferably from 90 to 150°C, and more preferably from 95 to 130°C.
The degree of vacuum of the aforementioned second drying treatment is preferably 13.3 kPa or less, more preferably 6.6 kPa or less, and still more preferably 3.9 kPa or less.
When the aforementioned temperature and the degree of vacuum are within the above-mentioned ranges, the water dispersibility will be satisfactory.

<Step (7)>

In the step (7), the slurry obtained in the aforementioned step (5) is neutralized by adding an acid thereto.
Examples of the acid include strong acids such as sulfuric acid, hydrochloric acid, and nitric acid, and weak acids such as acetic acid and phosphoric acid. Among these, hydrochloric acid, sulfuric acid and nitric acid are preferable due to their low cost.
With respect to the amount of acid to be used, the amount is such that pH following neutralization at 25°C when the final silane-modified cationized cellulose is prepared as an aqueous solution is preferably from 3.0 to 8.0, more preferably from 3.5 to 6.5. When the aforementioned pH is within the above-mentioned range, the water dispersibility of the silane-modified cationized cellulose that is ultimately obtained is satisfactory, and the solubility thereof in water is also favorable.

<Step (8)>

In the step (8), a first drying treatment in which the product obtained in the aforementioned step (7) is processed at a temperature of 50 to 120°C and a degree of vacuum of 13.4 to 53.3 kPa, and a second drying treatment in which the aforementioned product following the first drying treatment is processed at a temperature of 90 to 150°C and a degree of vacuum of 13.3 kPa or less are conducted.
The temperature of the aforementioned first drying treatment is preferably from 50 to 120°C, more preferably from 60 to 110°C, and still more preferably from 70 to 100°C.
The degree of vacuum of the aforementioned first drying treatment is preferably from 13.4 to 53.3 kPa, more preferably from 13.4 to 46.6 kPa, and still more preferably from 13.4 to 39.9 kPa.
When the aforementioned temperature and the degree of vacuum are within the above-mentioned ranges, the solvent removal efficiency and the color tone will be satisfactory.

<Step (9)>

In the step (9), a first drying treatment in which the product obtained in the aforementioned step (3) is processed at a temperature of 50 to 120°C and a degree of vacuum of 13.4 to 53.3 kPa, and a second drying treatment in which the aforementioned product following the first drying treatment is processed at a temperature of 90 to 150°C and a degree of vacuum of 13.3 kPa or less are conducted.
The temperature of the aforementioned first drying treatment is preferably from 50 to 120°C, more preferably from 60 to 110°C, and still more preferably from 70 to 100°C.
The degree of vacuum of the aforementioned first drying treatment is preferably from 13.4 to 53.3 kPa, more preferably from 13.4 to 46.6 kPa, and still more preferably from 13.4 to 39.9 kPa.
When the aforementioned temperature and the degree of vacuum are within the above-mentioned ranges, the solvent removal efficiency and the color tone will be satisfactory.
There are no particular limitations on the method of reacting cationized cellulose with an aminosilane compound (silane treatment method), and conventional silane treatment methods may be used depending on the purpose. However, in the present invention, following the step (3), it is necessary to conduct a silane treatment without bringing the cationized cellulose into contact with a solvent with a water content of 10% by weight or more (such as water and mixed solvents in which the proportion of water is 10% by weight or more).
As the cationized cellulose, in the aforementioned step (3), the slurry or cake in which water content of the solvent is adjusted to 10% by weight or less may be used as it is. Alternatively, a cake or dried product obtained by partially or completely removing the solvent of the aforementioned slurry or cake may also be used.
Preferred examples of the silane treatment methods include a method in which an aminosilane compound is added by atomization using a spray or the like to a slurry, cake or dried product of the aforementioned cationized cellulose to allow the reaction to proceed, followed by drying. In this case, in order to uniformly conduct a silane treatment, it is preferable to conduct stirring after addition of the aminosilane compound.
With respect to the silane treatment, there are no particular limitations on the reaction temperature when allowing the cationized cellulose to react with the aminosilane compound. The reaction temperature may be appropriately selected in accordance with the purpose, but is preferably from 20 to 80°C, more preferably from 25 to 75°C, and still more preferably from 30 to 70°C. When the aforementioned temperature is 20°C or higher, the reaction proceeds satisfactorily, and the water dispersibility of the obtained silane-modified cationized cellulose will be satisfactory. When the aforementioned temperature is 80°C or less, the color tone of the aforementioned silane-modified cationized cellulose will be satisfactory.
The reaction time is not particularly limited and may be appropriately selected in accordance with the reaction temperature, the purpose, and the like, but is preferably from 5 to 120 minutes, more preferably from 10 to 100 minutes, and still more preferably from 15 to 80 minutes. When the aforementioned reaction time is 5 minutes or more, the reaction proceeds satisfactorily, and the water dispersibility of the obtained silane-modified cationized cellulose will be satisfactory. When the aforementioned reaction time is within 120 minutes, the color tone of the aforementioned silane-modified cationized cellulose will be satisfactory.
With respect to drying of the reaction product obtained by the reaction of cationized cellulose with an aminosilane compound, for example, drying may be conducted by heating the aforementioned reaction product under reduced pressure or normal pressure. The heating temperature in this case is preferably within the range from 50 to 150°C, and more preferably within the range from 70 to 130°C, in consideration of the color tone of the dried product, the loss on drying (i.e., the liquid content) or the like.
It is also possible to conduct a deliquoring treatment of the aforementioned reaction product prior to the above-mentioned drying. The deliquoring treatment can be conducted, for example, by using a centrifugal deliquoring device which uses a filter cloth.
The silane-modified cationized cellulose produced by the above-mentioned production method exhibits excellent water dispersibility and disperses readily within a short period of time when introduced into water or aqueous solvents, such as a mixed solvent composed of water and a water-compatible organic solvent. Although the reasons why these effects are obtained remain unclear, the following reasons are thought to be responsible.
In the cationization of water-soluble cellulose, a large amount of water has been used conventionally either as a reaction solvent or for neutralization and washing of an alkali substance. For example, in the method described in Japanese Examined Patent Application, Second Publication No. Hei 7-72201, washing with a mixed solvent of 20% water/acetone for the removal of the neutralized salt produced by neutralization of alkali has been conducted following cationization. For this reason, the obtained cationized cellulose is swollen due to the above-mentioned water, and when a silane treatment is conducted on the cationized cellulose in such conditions, the silane treatment progresses not only on the surface but also even in the inside. As a result, it is thought that the process of making the surface hydrophobic was insufficient, and thus it was not possible to achieve an adequate level of water dispersibility.
On the other hand, in the above-mentioned production method, before conducting a silane treatment, a water-compatible

organic solvent or a mixed solvent composed of a water-compatible organic solvent and water is added to the cationized cellulose after the cationization treatment (which contains water) so that the water content in the entire solvent following the addition is 10% by weight or less. At this time, because the water-compatible organic solvent also mixes with the water contained in the cationized cellulose, the aforementioned water-compatible organic solvent with a low water content is brought into contact with the cationized cellulose, thereby extracting the water contained in the cationized cellulose. If the water content in the entire solvent including the water contained in the cationized cellulose is 10% by weight or less, the amount of water contained in the cationized cellulose would be reduced sufficiently. It is thought that the surface of cationized cellulose is sufficiently silane-modified and the water dispersibility improves by carrying out a silane treatment on the cationized cellulose, in which the water content is reduced in the above-mentioned manner, using a specific silane compound (aminosilane compound).

The form of the component (B) is not particularly limited and may be appropriately selected in accordance with the purpose. In consideration of the dispersibility and solubility in water or the like, a powder form is preferable.

When the component (B) is in a powder form, the particle size thereof may be appropriately selected in consideration of the intended purpose or the like, and is preferably from 10 to 1,000 μm, more preferably from 30 to 800 μm, and still more preferably from 50 to 600 μm. When the aforementioned particle size is 10 μm or more, the water dispersibility improves, and at the same time, dust is hardly produced during use, and the handling properties will be satisfactory. When the aforementioned particle size is 1,000 μm or less, the solubility in water will be satisfactory.

In addition, pH of the component (B) at 25°C when prepared as a 2% by weight aqueous solution is preferably from 5 to 7.5. When the aforementioned pH is 7.5 or less, the water dispersibility improves, whereas when the aforementioned pH is 5 or more, the solubility in water improves.

Either a single type of component (B) may be used alone, or two or more types thereof may be mixed for use. The content of the component (B) within the detergent composition, relative to the total weight of the detergent composition, is preferably from 0.001 to 4% by weight, more preferably from 0.01 to 3% by weight, and still more preferably from 0.02 to 2.5% by weight. When the content is at least as large as the lower limit of the aforementioned range, the effect of improving conditioning performance can be achieved satisfactorily, whereas if the content is equal to or less than the upper limit of the aforementioned range, stability with other components improves.

The detergent composition of the present invention may include a component other than the aforementioned components (A) and (B) as an optional component within a range that does not impair the effects of the present invention. The aforementioned optional component is not particularly limited, and various additive components that have been conventionally added to the detergent composition can be used.

Examples of the aforementioned additive components include nonionic surfactants, amphoteric surfactants, silicone compounds, liquid oil, solid fat, anionic polymers, nonionic polymers, cationic polymers (excluding those that correspond to the component (B)), polyols, inorganic salts (such as anhydrous sodium sulfate, common salt and salt cakes), organic salts, moisturizers (such as propylene glycol), tonic agents, solubilizing agents, antioxidants (such as dibutylhydroxytoluene (BHT) and α-tocopherol), bactericides (such as triclosan and trichlorocarban), viscosity modifiers (such as polymer compounds and fatty acid diethanolamide), ultraviolet absorbers, antioxidants, protein derivatives, plant and animal extracts, anti-dandruff agents (such as piroctone olamine and zinc pyrithione), anti-inflammatory drugs (such as dipotassium glycyrrhizate), preservatives (such as benzoic acid and the salts thereof, parabens and caisson CG), pH adjusters (such as citric acid and triethanolamine), hydrotropes, lower alcohols, vitamins, volatile oil, hydrophobic solvents, dilution solvents, pigments, perfumes, perfume compositions and the like. These additive components may be added alone or two or more types thereof may be mixed and added.

Examples of nonionic surfactants include monoethanolamide laurate, diethanolamine laurate, coconut oil fatty acid diethanolamide, polyoxyethylene alkyl phenyl ether, polyoxyethylene alkyl ether, polyoxyethylene hydrogenated castor oil and the like.

Examples of amphoteric surfactants include alkylamidopropylbetaines such as lauric acid amidopropylbetaine, alkyldimethylamine oxides such as lauryldimethylamine oxide, alkyldimethylcarboxymethyl ammonium betaine, alkylcarboxymethyl imidazolium betaine, N-(N'-acylaminoalkyl)-N-hydroxyalkylaminocarboxylates and the like.

There are no particular limitations on the type of silicone compounds, and those which are generally used in a shampoo composition can be used. Examples thereof include dimethylpolysiloxane (such as highly polymerized dimethylpolysiloxane and silicone rubber), methylphenylpolysiloxane, polyether-modified silicone, polyamino-modified silicone, betaine-modified silicone, alcohol-modified silicone, fluorine-modified silicone, epoxy-modified silicone, mercapto-modified silicone, carboxy-modified silicone, fatty acid-modified silicone, silicone graft polymers, cyclic silicone, alkyl-modified silicone, dimethylpolysiloxane with a trimethylsilyl terminal group, dimethylpolysiloxane with silanol terminal group, and the like. One type of these may be used alone, or two or more types thereof may be combined for use where appropriate.

Among these, dimethylpolysiloxane, polyether-modified silicone and polyamino-modified silicone are particularly suited for use.

Although there are no particular limitations on the viscosity or the like of these silicone compounds, in general, those exhibiting a viscosity (at a temperature of 25°C) from 1 to 20,000,000 $mm^2/s$, preferably from 30 to 1,000,000 $mm^2/s$

are favorably used. The method for measuring viscosity is in accordance with the "viscosity determination" specified in Japanese Standards of Cosmetic Ingredients.
As the above-mentioned silicone compound, those prepared as an emulsion by emulsifying the above-mentioned silicone derivatives with a surfactant can also be used. It should be noted that there are no particular limitations on the emulsifiers, nor on the emulsification methods, for preparing these emulsions, and various emulsifiers and emulsification methods can be employed.

Specific examples of anionic polymers and nonionic polymers include pectin, carageenan, guar gum, locust bean gum, gelatin, xanthan gum, carboxyvinyl polymers, carboxymethyl hydroxyethyl cellulose, hydroxyethyl cellulose, hydroxypropyl methylcellulose, alginates, starch, polyvinyl alcohols, polyacrylates, polymethacrylates, polymethylacrylic acids, polyethylene glycol, polyethylene oxid, tragacanth gum and the like.
Examples of cationic polymers include dimethyldiallyl ammonium chloride homopolymers having dimethyldiallyl ammonium halide as a functional group, cationized cellulose, cationized guar gum, cationized dextran, cationized pullulan, quaternized vinyl pyrrolidone/aminoethyl methacrylate copolymers, polyethyleneimine, dipropylenetriamine condensates, dimethyl adipate/aminohydroxypropyldiethyl triamine copolymers, quaternary nitrogen-containing starch and the like, as well as those hydrolyzed proteins to which a cationic group has been introduced, such as cationized hydrolyzed keratin, cationized hydrolyzed silk, cationized hydrolyzed collagen, cationized hydrolyzed wheat, siliconized hydrolyzed collagen, siliconized hydrolyzed silk and the like.
One type of these polymers can be used alone or two or more types thereof can be suitably used in combination where appropriate.

Examples of perfumes and perfume compositions include perfume components and the like described in paragraphs [0021] to [0035] in Japanese Laid-Open Patent Application No. 2003-300811, and solvents for perfumes and the like described in paragraph [0050] in the same publication. Here, a perfume composition refers to a mixture constituted of the aforementioned perfume component, a solvent, a perfume stabilizer and the like.
In the perfume composition, the solvent for perfumes is typically added within a range from 0.1 to 99% by weight, preferably from 1 to 50% by weight.
Examples of perfume stabilizers include BHT, butylhydroxyanisole, vitamin E and the derivatives thereof, catechin compounds, flavonoid compounds, polyphenol compounds and the like. Of these, examples of preferred stabilizers include dibutylhydroxytoluene.
In the perfume composition, the perfume stabilizer is typically added within a range from 0.0001 to 10% by weight, preferably from 0.001 to 5% by weight.
In those cases where such a perfume composition is added to the detergent composition, the aforementioned perfume composition is typically added within a range from 0.005 to 40% by weight, preferably from 0.01 to 10% by weight, relative to the total amount of the detergent composition.

The detergent composition of the present invention can be prepared in accordance with ordinary methods. For example, it can be produced by mixing and stirring the above-mentioned components (A) and (B), optional components and water (balance). During this process, the component (A) can be readily produced by using an aqueous solution of anionic surfactant which has been diluted with water in advance and having a concentration of 20 to 70% by weight. The component (B) can be suitably added by a method to dissolve or disperse in water or the like in advance or to disperse in a poor solvent such as propylene glycol.

The detergent composition of the present invention preferably has a viscosity (at 25°C) from 100 to 5,000 mPa·s and more preferably from 500 to 3,000 mPa·s. When the viscosity is at least as large as the lower limit of the above-mentioned range, handling properties are favorable. Also when the viscosity is not higher than the upper limit of the above-mentioned range, handling properties are favorable.

The detergent composition of the present invention preferably has a pH (at 25°C) from 3 to 10 and more preferably from 5 to 7. If the pH is at least as high as the lower limit of the above-mentioned range, the level of skin irritation can be reduced. On the other hand, if the pH is not higher than the upper limit, stability or the like is favorable.

[Examples]

The present invention will be described below in more detail using a series of examples.
In the following examples, the terms "parts" and "%" refer to "parts by weight" and "% by weight", respectively, unless specifically stated otherwise.

The materials and analytical methods used in the following Production Examples 1 to 6 are as follows.

<Materials>

* Hydroxyethyl cellulose:

AH-15L manufactured by Sumitomo Seika Chemicals Co., Ltd.; purity: 80%; viscosity of 2% by weight aqueous solution (25°C): 1,200 mPa·s
AX-15 manufactured by Sumitomo Seika Chemicals Co., Ltd.; purity: 80%; viscosity of 2% by weight aqueous solution (25°C): 26,000 mPa·s
LF-15: manufactured by Sumitomo Seika Chemicals Co., Ltd.; purity: 80%; viscosity of 2% by weight aqueous solution (25°C): 1,200 mPa·s
SH-15: manufactured by Sumitomo Seika Chemicals Co., Ltd.; purity: 80%; viscosity of 2% by weight aqueous solution (25°C): 1,200 mPa·s
SY-25: manufactured by Sumitomo Seika Chemicals Co., Ltd.; purity: 90%; viscosity of 1 % by weight aqueous solution (25°C): 3,200 mPa·s

* Isopropyl alcohol: manufactured by Kanto Chemical Co., Inc.; purity: 99.5%
* Sodium hydroxide: manufactured by Kanto Chemical Co., Inc.
* Glycidyl trimethyl ammonium chloride: SY-GTA80 manufactured by Sakamoto Yakuhin Kogyo Co., Ltd.; effective concentration: 73% aqueous solution
* 3-aminopropyltriethoxysilane: KBE-903 manufactured by Shin-Etsu Chemical Co., Ltd.; effective concentration: 100%

<Analytical method>

[Water content (%) (Karl Fischer method)]

0.3 g of samples were provided for the analysis using the AQV-7 Trace Water Measuring Device manufactured by Hiranuma Sangyo Corporation.

[Amount of treating silane (%)]

The amount of silane used in the treatment was calculated by the following equation.

$$(\text{Amount of treating silane}) = (B/A) \times 100\ (\%)$$

[In the equation, A represents an amount of effective addition (total weight (g) $\times$ purity (%)) of hydroxyethyl cellulose and B represents an amount of effective addition (total weight (g) $\times$ effective concentration (%)) of 3-aminopropyltriethoxysilane.] [pH]
pH was measured using the pH meter PH71 manufactured by Yokogawa Electric Corporation.

[Production Example 1]

50 g (100 parts by weight) of a hydroxyethyl cellulose (AH-15L), 500 g (1,000 parts by weight) of a mixed solvent containing isopropyl alcohol/water (weight ratio) = 85/15 and 7.1 g (14.2 parts by weight) of a 25% by weight sodium hydroxide aqueous solution were placed in a separable flask and stirred and mixed for 30 minutes. Thereafter, stirring was stopped to produce a stationary condition, and 250 g (500 parts by weight) of the supernatant of the mixed solvent was removed. Subsequently, the temperature of the remainder of the separable flask was raised to 50°C, 24 g (48 parts by weight) of glycidyl trimethyl ammonium chloride was added thereto as a cationizing agent, and stirring of the resulting mixture was continued for 3 hours at 50°C. Thereafter, the pH was adjusted to 4.5 (50°C) by adding a 10% by weight isopropyl alcohol hydrochloride solution to obtain a cationized cellulose slurry.
Subsequently, 550 g of isopropyl alcohol (99.5%) was added to this slurry, and the resulting mixture was stirred and mixed for 15 minutes. Thereafter, stirring was stopped to produce a stationary condition, and 600 g of the supernatant thereof was removed. Water content of this supernatant was 7%.
Subsequently, this slurry was heated to 50°C, 1.2 g (2.4 parts by weight, amount of treating silane: 3%) of 3-aminopropyltriethoxysilane was added thereto and mixed to allow the reaction to proceed for 45 minutes. Then, this slurry underwent deliquoring using a centrifugal dehydrator, and drying (105°C) under reduced pressure was conducted for 5 hours, thereby obtaining a powder of the target silane-modified cationized cellulose.
The pH (at 25°C) of a 2% aqueous solution of the thus obtained silane-modified cationized cellulose was 7.0, and the

viscosity thereof (at 25°C) was 350 mPa·s.

[Production Example 2]

50 g (100 parts by weight) of a hydroxyethyl cellulose (AX-15), 500 g (1,000 parts by weight) of a mixed solvent containing isopropyl alcohol/water (weight ratio) = 85/15 and 7.1 g (14.2 parts by weight) of a 25% by weight sodium hydroxide aqueous solution were placed in a separable flask and stirred and mixed for 30 minutes. Thereafter, stirring was stopped to produce a stationary condition, and 250 g (500 parts by weight) of the supernatant of the mixed solvent was removed. Subsequently, the temperature of the remainder of the separable flask was raised to 50°C, 24 g (48 parts by weight) of glycidyl trimethyl ammonium chloride was added thereto as a cationizing agent, and stirring of the resulting mixture was continued for 3 hours at 50°C. Thereafter, the pH was adjusted to 4.5 (50°C) by adding a 10% by weight isopropyl alcohol hydrochloride solution to obtain a cationized cellulose slurry.
Subsequently, 550 g of isopropyl alcohol (99.5%) was added to this slurry, and the resulting mixture was stirred and mixed for 15 minutes. Thereafter, stirring was stopped to produce a stationary condition, and 600 g of the supernatant thereof was removed. Water content of this supernatant was 7%.
Subsequently, this slurry was heated to 50°C, 1.2 g (2.4 parts by weight, amount of treating silane: 3%) of 3-aminopropyltriethoxysilane was added thereto and mixed to allow the reaction to proceed for 45 minutes. Then, this slurry underwent deliquoring using a centrifugal dehydrator, and drying (105°C) under reduced pressure was conducted for 1.5 hours, thereby obtaining a powder of the target silane-modified cationized cellulose.
The pH (at 25°C) of a 2% aqueous solution of the thus obtained silane-modified cationized cellulose was 7.0, and the viscosity (at 25°C) of the 1% aqueous solution thereof was 900 mPa·s.

[Production Example 3]

30 g (100 parts by weight) of a hydroxyethyl cellulose (LF-15), 300 g (1,000 parts by weight) of a mixed solvent containing isopropyl alcohol/water (weight ratio) = 85/15 and 4.5 g (15 parts by weight) of a 25% by weight sodium hydroxide aqueous solution were placed in a separable flask and stirred and mixed for 30 minutes. Thereafter, stirring was stopped to produce a stationary condition, and 150 g (500 parts by weight) of the supernatant of the mixed solvent was removed. Subsequently, the temperature of the remainder of the separable flask was raised to 50°C, 15 g (50 parts by weight) of glycidyl trimethyl ammonium chloride was added thereto as a cationizing agent, and stirring of the resulting mixture was continued for 3 hours at 50°C. Thereafter, the pH was adjusted to 6 (50°C) by adding a 10% by weight isopropyl alcohol hydrochloride solution to obtain a cationized cellulose slurry. When water content of this supernatant was measured, it was 16%.
Subsequently, while retaining the above-mentioned slurry at 50°C, 0.6 g (2 parts by weight, amount of treating silane: 2.5%) of 3-aminopropyltriethoxysilane was added thereto and mixed to allow the reaction to proceed for 30 minutes.
Then, this slurry underwent deliquoring using a centrifugal dehydrator, and the obtained cake was dried at 80°C, thereby obtaining a powder of the target silane-modified cationized cellulose.
The pH (at 25°C) of a 2% aqueous solution of the thus obtained silane-modified cationized cellulose was 6.5, and the viscosity thereof (at 25°C) was 340 mPa·s.

[Production Example 4]

To 30 g (100 parts by weight) of a hydroxyethyl cellulose (manufactured by Sumitomo Seika Chemicals Co., Ltd., product name: SH-15, viscosity of 2% by weight aqueous solution (25°C): 1,200 mPa·s) were added and mixed 120 g (400 parts by weight) of a mixed solvent containing isopropyl alcohol (IPA)/water (weight ratio) = 85/15, and 1.8 g (6 parts by weight) of a 25% by weight aqueous solution of sodium hydroxide. Subsequently, the temperature was raised to 50°C, 15.8 g (53 parts by weight) of glycidyl trimethyl ammonium chloride (manufactured by Sakamoto Yakuhin Kogyo Co., Ltd., product name: SY-GTA80, effective concentration: 73% by weight aqueous solution) was added as a cationizing agent, and cationization was conducted by allowing the reaction to proceed for 3 hours. pH of the reaction slurry during this process was 12. 0.6 g (2 parts by weight) of 3-aminopropyltriethoxysilane (KBE-903 manufactured by Shin-Etsu Chemical Co., Ltd.; effective concentration: 100%) was added thereto as an aminosilane-based coupling agent and mixed, and a treatment was conducted at 50°C for 45 minutes. Thereafter, the pH was adjusted to 5 by adding a 10% by weight IPA hydrochloride solution to obtain a silane-modified cationized cellulose slurry. The aforementioned silane-modified cationized cellulose slurry was washed with 1,350 g (4,500 parts by weight) of a mixed solvent containing isopropyl alcohol (IPA)/water (weight ratio) = 85/15, followed by deliquoring, and the obtained cake (solid content of 60%) was subjected to a first drying treatment at a temperature of 80°C and a degree of vacuum of 20.0 kPa (150 Torr) for 1 hour. Thereafter, by conducting a second drying treatment at a temperature of 120°C and a degree of vacuum of 1.3 kPa (10 Torr) for 1 hour, a powder of the target silane-modified cationized cellulose was obtained.

In Production Example 4, the amount of residual IPA at the time of termination of the first drying treatment was lower than the detection limit (hereafter, expressed as N.D.), the loss on drying at the time of termination of the first drying treatment was 18.4%, and the loss on drying at the time of termination of the second drying treatment was 0.5%.

[Production Example 5]

The same operation as in Production Example 4 was conducted with the exception that the amount of glycidyl trimethyl ammonium chloride added was changed to 7.6 g (25.2 parts by weight) and the amount of 3-aminopropyltriethoxysilane added was changed to 0.9 g (3 parts by weight), thereby obtaining a powder of the target silane-modified cationized cellulose.
In Production Example 5, the amount of residual IPA at the time of termination of the first drying treatment was 0.004%, the loss on drying at the time of termination of the first drying treatment was 15.4%, and the loss on drying at the time of termination of the second drying treatment was 0.5%.

[Production Example 6]

To 30 g (100 parts by weight) of a hydroxyethyl cellulose (manufactured by Sumitomo Seika Chemicals Co., Ltd., product name: SY-25, viscosity of 1% by weight aqueous solution (25°C): 3,200 mPa·s) were added and mixed 120 g (400 parts by weight) of a mixed solvent containing isopropyl alcohol (IPA)/water (weight ratio) = 85/15, and 1.8 g (6 parts by weight) of a 25% by weight aqueous solution of sodium hydroxide. Subsequently, the temperature was raised to 50°C, 15.8 g (53 parts by weight) of glycidyl trimethyl ammonium chloride (manufactured by Sakamoto Yakuhin Kogyo Co., Ltd., product name: SY-GTA80, effective concentration: 73% by weight aqueous solution) was added thereto as a cationizing agent, and cationization was conducted by allowing the reaction to proceed for 3 hours. pH of the reaction slurry during this process was 12. 2 g (6.5 parts by weight) of 3-aminopropyltriethoxysilane (KBE-903 manufactured by Shin-Etsu Chemical Co., Ltd.; effective concentration: 100%) was added thereto as an aminosilane-based coupling agent and mixed, and a treatment was conducted at 50°C for 45 minutes. Thereafter, the pH was adjusted to 5 by adding a 10% by weight IPA hydrochloride solution to obtain a silane-modified cationized cellulose slurry. The aforementioned silane-modified cationized cellulose slurry was washed with 1,350 g (4,500 parts by weight) of a mixed solvent containing isopropyl alcohol (IPA)/water (weight ratio) = 85/15, followed by deliquoring, and the obtained cake (solid content of 60%) was subjected to a first drying treatment at a temperature of 80°C and a degree of vacuum of 20.0 kPa (150 Torr) for 1 hour. Thereafter, by conducting a second drying treatment at a temperature of 105°C and a degree of vacuum of 2.0 kPa (15 Torr) for 1 hour, a powder of the target silane-modified cationized cellulose was obtained.
In Production Example 6, the amount of residual IPA at the time of termination of the first drying treatment was lower than the detection limit, the loss on drying at the time of termination of the first drying treatment was 18.2%, and the loss on drying at the time of termination of the second drying treatment was 0.6%.
The powders of silane-modified cationized cellulose obtained in Production Examples 1 to 6 were fractionated using a sieve, and those obtained with a particle size of 106 to 425 μm were used as samples to conduct the following evaluations. The results are shown in Tables 1 and 2.

[Water dispersibility]

(i) Amount of dispersion after 2 minutes:

50 g of distilled water was added to a 100 mL beaker, and a sample of 0.5 g was introduced from a height of 4 cm above the water surface. Two minutes after introducing the sample, the proportion (%) of powder that dispersed into the water and did not remain on the water surface was evaluated by visual observation, and this was defined as the amount of dispersion after two minutes.

(ii) Time required for complete dispersion:

50 g of distilled water was added to a 100 mL beaker, and a sample of 0.5 g was introduced from a height of 4 cm above the water surface. After completing the introduction of the sample, the time required until the powder completely dispersed in the water was measured, and this was defined as the time required for complete dispersion.

It should be noted that with respect to the powders which did not completely disperse even after 300 seconds, measurement was terminated at that time point, and an evaluation of "300 seconds or more" was given thereto.

[Table 1]

| | | Production Example 1 | Production Example 2 | Production Example 3 |
|---|---|---|---|---|
| Water dispersibility | Amount of dispersion after 2 minutes (%) | 100 | 100 | 100 |
| | Time required for complete dispersion (seconds) | 16 | 17 | 25 |

[Table 2]

| | | Production Example 4 | Production Example 5 | Production Example 6 |
|---|---|---|---|---|
| Water dispersibility | Amount of dispersion after 2 minutes (%) | 100 | 100 | 100 |
| | Time required for complete dispersion (seconds) | 10 | 12 | 23 |

As is apparent from the above-mentioned results, the samples obtained in Production Examples 1, 2, 4 and 5 resulted in shorter time required for complete dispersion and thus superior water dispersibility, as compared to the samples obtained in Production Examples 3 and 6.

[Examples 1 to 4 and 9 to 12, Comparative Examples 1 to 3]

Hair detergent compositions were prepared based on the compositions (%) indicated in Tables 3 and 5. The following evaluations were conducted for each hair detergent composition. The results of the evaluations are summarized in Tables 3 and 5.

<Evaluation method>

[Determination of amount of precipitated complex]

With respect to the hair detergent compositions, it has been known that cationic polymers, such as cationized celluloses, form complexes and precipitate with surfactants serving as a cleaning component when diluted with water, and that there is a positive correlation between this amount of precipitated complexes and the conditioning performance. Accordingly, the amount of precipitated complexes was determined by the following procedures as one of the indicators to evaluate the conditioning performance.
A hair detergent composition was charged into a centrifuge tube which was weighed to a constant weight, and distilled water was added thereto so as to achieve the respective dilution rates indicated in Table 2, followed by a treatment for 30 minutes using an ultrasonic cleaner. Thereafter, a centrifugal separation treatment was carried out at 3,000 rpm for 30 minutes using a centrifugal separator, thereby precipitating complexes. Then, the supernatant was removed and the resultant was dried at 105°C for 3 hours. The weight of the obtained residue was measured, and an amount of precipitated complexes (mg/g) per 1 g of the used hair detergent composition was determined from this measured value.

[Rinsing properties]

Shampooing was carried out by 10 panelists using each hair detergent composition and a sensory evaluation was conducted with respect to the friction during rinsing. In the sensory evaluation, rating was conducted based on the following sensory evaluation criteria, and the average value was determined.

(Sensory evaluation criteria)

5 points: Almost no sense of friction during rinsing
4 points: Somewhat feels a sense of friction during rinsing
3 points: Feels a sense of friction during rinsing
2 points: Feels a strong sense of friction during rinsing
1 point: Feels a strong sense of friction during rinsing which hinders finger combing

Rinsing properties of each hair detergent composition were evaluated from the average values determined as described above, based on the following criteria.

(Criteria)

A: 4 points or more
B: 3 points or more and less than 4 points
C: 2 points or more and less than 3 points
D: less than 2 points

[Examples 5 to 8 and 13 and 16, Comparative Examples 4 to 6]

Skin detergent compositions were prepared based on the compositions (%) indicated in Tables 4 and 6. The following evaluations were conducted for each skin detergent composition. The results of the evaluations are summarized in Tables 4 and 6.

<Evaluation method>

[Measurement of water recovery rate]

It has been known that the moisture on the skin surface is lost during washing of the body due to the cleaning component. For this reason, the water recovery rate has been used as an evaluation indicator for the conditioning performance. In addition, it has been known that there is a positive correlation between the water retention and moist feeling. Accordingly, the water recovery rate was measured by the following procedures as one of the indicators to evaluate the conditioning performance.

1. Measuring device

Horny layer moisture content: 3.5 MHz high frequency conductivity measuring device SKICON 200 (manufactured by I.B.S Co., Ltd.)

2. Measurement of impedance before washing

After washing the arm portion to be evaluated with tap water for 1 minute, moisture was wiped away with a towel. Thereafter, it was left in a stationary position in a thermostatic chamber (25°C - 60%) for 30 minutes, and the impedance before washing was measured. Note that data is an average of N = 5.

3. Measurement of impedance after washing

A cup holder (diameter of 4 cm × height of 1.5 cm) used for washing was attached to the arm portion, and 5

mL of a detergent composition diluted with water at 40°C by 20-fold was added thereto from the upper portion and shaken for 5 minutes on a shaker. Thereafter, the detergent composition was removed from the upper portion and washing was repeated 6 times using 5 mL of water at 40°C. The cup was removed, and moisture was removed by gently laying a towel. Thereafter, it was left in a stationary position in a thermostatic chamber (25°C - 60%) for 30 minutes, and the impedance after washing was measured. Note that data is an average of N = 5.
The water recovery rate was calculated by the following equation.

$$\text{Water recovery rate (\%)} = (\text{impedance } (\Omega) \text{ after washing}) / (\text{impedance } (\Omega) \text{ before washing}) \times 100$$

[Moist feeling]

Body washing was carried out by 10 panelists using each skin detergent composition and a sensory evaluation was conducted with respect to the moist feeling 3 days after the washing. In the sensory evaluation, rating was conducted based on the following sensory evaluation criteria, and the average value was determined.

(Sensory evaluation criteria)

5 points: Feels a strong sense of moist feeling after washing

4 points: Feels a somewhat strong sense of moist feeling after washing

3 points: Feels a sense of moist feeling after washing

2 points: Slightly feels a sense of moist feeling after washing

1 point: Hardly feels a sense of moist feeling after washing

Moist feeling of each skin detergent composition was evaluated from the average value determined as described above, based on the following criteria.

(Criteria)

A: 4 points or more

B: 3 points or more and less than 4 points

C: 2 points or more and less than 3 points

D: less than 2 points

[Table 3]

| Composition | | Ex. 1 | Ex. 2 | Ex. 3 | Ex. 4 | Comp. Ex. 1 | Comp. Ex. 2 | Comp. Ex.3 |
|---|---|---|---|---|---|---|---|---|
| Cleaning component | Sodium polyoxyethylene alkyl ether sulfate | 12 | 12 | 12 | 12 | 12 | 12 | 12 |
| Conditioning component | Cationized cellulose (1) | 0.7 | 1.0 | | | | | |
| | Cationized cellulose (2) | | | 0.7 | | | | |
| | Cationized cellulose (3) | | | | 0.7 | | | |
| | Cationized cellulose (4) | | | | | 0.7 | | 0.7 |
| Auxiliary | 3-aminopropyl-triethoxysilane | | | | | | | 5.0 |
| Inorganic salt | Anhydrous sodium sulfate | 1.0 | 1.0 | 1.0 | 1.0 | 1.0 | 1.0 | 1.0 |
| pH adjuster | Citric acid | Adequate amount | Adequate amount | Adequate amount | Adequate amount | Adequate amount | Adequate amount | Adequate amount |
| Water | | Balance | Balance | Balance | Balance | Balance | Balance | Balance |
| Evaluation | Dilution rate | 3 | 3 | 3 | 3 | 3 | 3 | 3 |
| | Amount of precipitated complex (mg/g) | 16.4 | 28.2 | 25.1 | 15.2 | 12.9 | N.D. | 12.5 |
| | Rinsing properties | B | A | A | B | C | D | C |
| N.D.: Lower than detection limit | | | | | | | | |

[Table 4]

| Composition | | Ex. 5 | Ex. 6 | Ex. 7 | Ex. 8 | Comp. Ex. 4 | Comp. Ex. 5 | Comp. Ex. 6 |
|---|---|---|---|---|---|---|---|---|
| Base material | Lauric acid | 6.7 | 6.7 | 6.7 | 6.7 | 6.7 | 6.7 | 6.7 |
| | Myristic acid | 9.4 | 9.4 | 9.4 | 9.4 | 9.4 | 9.4 | 9.4 |
| Stabilizer | Polypropylene glycol | 9.0 | 9.0 | 9.0 | 9.0 | 9.0 | 9.0 | 9.0 |
| Conditioning component | Cationized cellulose (1) | 0.05 | 1.0 | | | | | |
| | Cationized cellulose (2) | | | 0.05 | | | | |
| | Cationized cellulose (3) | | | | 0.05 | | | |
| | Cationized cellulose (4) | | | | | 0.05 | | 0.05 |
| Auxiliary | 3-aminopropyl-triethoxysilane | | | | | | | 5.0 |
| Neutralizer | 48% potassium hydroxide | 4.2 | 4.2 | 4.2 | 4.2 | 4.2 | 4.2 | 4.2 |
| Water | | Balance | Balance | Balance | Balance | Balance | Balance | Balance |
| Evaluation | Water recovery rate (%) | 88 | 93 | 85 | 82 | 55 | 42 | 45 |
| | Moist feeling | B | A | B | B | C | D | D |

[Table 5]

| Composition | | Ex. 9 | Ex. 10 | Ex. 11 | Hex. 12 |
|---|---|---|---|---|---|
| Cleaning component | Sodium polyoxyethylene alkyl ether sulfate | 12 | 12 | 12 | 12 |
| Conditioning component | Cationized cellulose (5) | 0.7 | 1.0 | | |
| | Cationized cellulose (6) | | | 0.7 | |
| | Cationized cellulose (7) | | | | 0.7 |
| | Cationized cellulose (4) | | | | |
| Auxiliary | 3-aminopropyltriethoxysilane | | | | |
| Inorganic salt | Anhydrous sodium sulfate | 1.0 | 1.0 | 1.0 | 1.0 |
| pH adjuster | Citric acid | Adequate amount | Adequate amount | Adequate amount | Adequate amount |
| Water | | Balance | Balance | Balance | Balance |

(continued)

| Composition | | Ex. 9 | Ex. 10 | Ex. 11 | Hex. 12 |
|---|---|---|---|---|---|
| Evaluation | Dilution rate | 3 | 3 | 3 | 3 |
| | Amount of precipitated complex (mg/g) | 15.2 | 29.4 | 26.8 | 16.1 |
| | Rinsing properties | B | A | A | B |

[Table 6]

| Composition | | Ex. 13 | Ex.14 | Ex. 15 | Ex. 16 |
|---|---|---|---|---|---|
| Base material | Lauric acid | 6.7 | 6.7 | 6.7 | 6.7 |
| | Myristic acid | 9.4 | 9.4 | 9.4 | 9.4 |
| Stabilizer | Polypropylene glycol | 9.0 | 9.0 | 9.0 | 9.0 |
| Conditioning component | Cationized cellulose (5) | 0.05 | 1.0 | | |
| | Cationized cellulose (6) | | | 0.05 | |
| | Cationized cellulose (7) | | | | 0.05 |
| | Cationized cellulose (4) | | | | |
| Auxiliary | 3-aminopropyltriethoxysilane | | | | |
| Neutralizer | 48% potassium hydroxide | 4.2 | 4.2 | 4.2 | 4.2 |
| Water | | Balance | Balance | Balance | Balance |
| Evaluation | Water recovery rate (%) | 87 | 95 | 82 | 84 |
| | Moist feeling | B | A | B | B |

In Tables 3 to 6, units of the added amounts of each component are %. The expression "adequate amount" for the pH adjuster indicate an amount so as to achieve an ultimate pH (at 25°C) of hair detergent composition of 6.0. The pH of hair detergent compositions was measured using the pH meter PH71 manufactured by Yokogawa Electric Corporation.
The expression "balance" means a certain amount of water was added so as to achieve a total of 100%.
In addition, the components used herein are as follows.

- Sodium polyoxyethylene alkyl ether sulfate: Sinolin SP1350 manufactured by New Japan Chemical Co., Ltd.; number of moles of added ethylene oxide (E.O.): 3 moles; number of carbon atoms of alkyl group: 8 to 20
- Cationized cellulose (1): Silane-modified cationized cellulose obtained in Production Example 1 (viscosity of 2% aqueous solution (25°C): 350 mPa·s, degree of cationization: 1.8 wt.%)
- Cationized cellulose (2): Silane-modified cationized cellulose obtained in Production Example 2 (viscosity of 1% aqueous solution (25°C): 900 mPa·s, degree of cationization: 1.8 wt.%)
- Cationized cellulose (3): Silane-modified cationized cellulose obtained in Production Example 3 (viscosity of 2% aqueous solution (25°C): 340 mPa·s, degree of cationization: 1.1 wt.%)
- Cationized cellulose (4): Glyoxal-treated cationized cellulose (Leoguard GP (manufactured by Lion Corporation), viscosity of 2% aqueous solution (25°C): 340 mPa·s, degree of cationization: 1.8 wt.%)
- 3-aminopropyltriethoxysilane: KBE-903 manufactured by Shin-Etsu Chemical Co., Ltd. (effective concentration: 100%)
- Cationized cellulose (5): Silane-modified cationized cellulose obtained in Production Example 4 (viscosity of 2% aqueous solution (25°C): 370 mPa·s, degree of cationization: 1.8 wt.%)
- Cationized cellulose (6): Silane-modified cationized cellulose obtained in Production Example 5 (viscosity of 2% aqueous solution (25°C): 450 mPa·s, degree of cationization: 1.1 wt.%)
- Cationized cellulose (7): Silane-modified cationized cellulose obtained in Production Example 6 (viscosity of 1% aqueous solution (25°C): 950 mPa·s, degree of cationization: 1.8 wt.%)

As shown in the above-mentioned results, the hair detergent compositions of Examples 1 to 4 and 9 to 12 produced a large amount of precipitated complexes which contributes to the conditioning performance, and the sensory evaluation results thereof for rinsing properties were also favorable.
On the other hand, in Comparative Example 1 in which a glyoxal-treated cationized cellulose was used, only a small amount of precipitated complexes was produced, and the rinsing properties were also poor. In Comparative Example 2 in which no cationized cellulose was added, complexes did not precipitate and the rinsing properties were also poor. In Comparative Example 3 in which 3-aminopropyltriethoxysilane was added to the same composition as that of Comparative Example 1, although the amount of precipitated complexes was slightly increased, the rinsing properties were even worse than those in Comparative Example 1.

The skin detergent compositions of Examples 5 to 8 and 13 to 16 exhibited high levels of water recovery rate which contribute to the conditioning performance, and the sensory evaluation results thereof for moist feeling were also favorable.
On the other hand, in Comparative Example 4 in which a glyoxal-treated cationized cellulose was used as a cationized cellulose, the water recovery rate was low, and the sensory evaluation result thereof for moist feeling was also poor. In Comparative Example 5 in which no cationized cellulose was added, the water recovery rate was even lower, and the sensory evaluation result thereof for moist feeling was also poor. In Comparative Example 6 in which 3-aminopropyltriethoxysilane was added to the same composition as that of Comparative Example 4, the water recovery rate was lower than that of Comparative Example 4, and the sensory evaluation result thereof for moist feeling was also poor.

INDUSTRIAL APPLICABILITY

According to the present invention, a detergent composition that exhibits excellent conditioning performance can be provided, and thus can be used in hair detergents, skin detergents, and the like.

**Claims**

**1.** A hair detergent composition comprising:

an anionic surfactant; and
a silane-modified cationized cellulose.

**2.** The hair detergent composition according to claim 1, wherein the anionic surfactant is a polyoxyethylene alkyl ether sulfate.

**3.** The hair detergent composition according to claim 1 or 2,
wherein the silane-modified cationized cellulose is produced by a production method that comprises the following steps (1) to (4):

step (1): a step of cationizing a water-soluble cellulose ether in a mixed solvent composed of a water-compatible organic solvent and water under the presence of an alkali, thereby obtaining a slurry that contains a cationized cellulose;
step (2): a step of adding an acid to the slurry obtained in the step (1) to neutralize the alkali;
step (3): a step of adding a water-compatible organic solvent or a mixed solvent composed of a water-compatible organic solvent and water so that, with respect to the cationized cellulose following neutralization, a water content in an entire solvent that comes into contact with the cationized cellulose becomes 10% by weight or less; and
step (4): a step of reacting the cationized cellulose obtained in the step (3) with an aminosilane compound.

**4.** The hair detergent composition according to claim 1 or 2,
wherein the silane-modified cationized cellulose is produced by a production method that comprises the following steps (1) and (5):

step (1): a step of cationizing a water-soluble cellulose ether in a mixed solvent composed of a water-compatible organic solvent and water under the presence of an alkali, thereby obtaining a slurry that contains a cationized cellulose; and
step (5): a step of adding an aminosilane compound to the slurry obtained in the step (1) or a cake thereof, thereby treating the cationized cellulose with the aminosilane compound,

the step wherein an amount of the aminosilane compound added is from 0.05 to 20% by weight relative to the water-soluble cellulose ether, and a treatment of the cationized cellulose with the aminosilane compound is conducted by a reaction under alkaline conditions at a pH of 10 or more.

**5.** The hair detergent composition according to claim 4,
wherein the silane-modified cationized cellulose is produced by a production method that further comprises the following step (6):

step (6): a step of conducting a first drying treatment in which a product obtained in the step (5) is processed at a temperature of 50 to 120°C and a degree of vacuum of 13.4 to 53.3 kPa, and a second drying treatment in which the product following the first drying treatment is processed at a temperature of 90 to 150°C and a degree of vacuum of 13.3 kPa or less.

**6.** The hair detergent composition according to claim 4,
wherein the silane-modified cationized cellulose is produced by a production method that further comprises the following steps (7) and (8):

step (7): a step of adding an acid to a reaction product obtained in the step (5) for neutralization; and
step (8): a step of conducting a first drying treatment in which a product obtained in the step (7) is processed at a temperature of 50 to 120°C and a degree of vacuum of 13.4 to 53.3 kPa, and a second drying treatment in which the product following the first drying treatment is processed at a temperature of 90 to 150°C and a degree of vacuum of 13.3 kPa or less.

**7.** The hair detergent composition according to claim 4,
wherein the silane-modified cationized cellulose is produced by a production method that further comprises the following steps (7), (3) and (9):

step (7): a step of adding an acid to a reaction product obtained in the step (5) for neutralization;
step (3): a step of adding a water-compatible organic solvent or a mixed solvent composed of a water-compatible organic solvent and water so that, with respect to the cationized cellulose following neutralization, a water content in an entire solvent that comes into contact with the cationized cellulose becomes 10% by weight or less; and
step (9): a step of conducting a first drying treatment in which a product obtained in the step (3) is processed at a temperature of 50 to 120°C and a degree of vacuum of 13.4 to 53.3 kPa, and a second drying treatment in which the product following the first drying treatment is processed at a temperature of 90 to 150°C and a degree of vacuum of 13.3 kPa or less.

**8.** A skin detergent composition comprising:

an anionic surfactant; and
a silane-modified cationized cellulose.

**9.** The skin detergent composition according to claim 8,
wherein the silane-modified cationized cellulose is produced by a production method that comprises the following steps (1) to (4):

step (1): a step of cationizing a water-soluble cellulose ether in a mixed solvent composed of a water-compatible organic solvent and water under the presence of an alkali, thereby obtaining a slurry that contains a cationized cellulose;
step (2): a step of adding an acid to the slurry obtained in the step (1) to neutralize the alkali;
step (3): a step of adding a water-compatible organic solvent or a mixed solvent composed of a water-compatible organic solvent and water so that, with respect to the cationized cellulose following neutralization, a water content in an entire solvent that comes into contact with the cationized cellulose becomes 10% by weight or less; and
step (4): a step of reacting the cationized cellulose obtained in the step (3) with an aminosilane compound.

**10.** The skin detergent composition according to claim 8,
wherein the silane-modified cationized cellulose is produced by a production method that comprises the following

steps (1) and (5):

step (1): a step of cationizing a water-soluble cellulose ether in a mixed solvent composed of a water-compatible organic solvent and water under the presence of an alkali, thereby obtaining a slurry that contains a cationized cellulose; and
step (5): a step of adding an aminosilane compound to the slurry obtained in the step (1) or a cake thereof, thereby treating the cationized cellulose with the aminosilane compound,
the step wherein an amount of the aminosilane compound added is from 0.05 to 20% by weight relative to the water-soluble cellulose ether, and a treatment of the cationized cellulose with the aminosilane compound is conducted by a reaction under alkaline conditions at a pH of 10 or more.

**11.** The skin detergent composition according to claim 10,
wherein the silane-modified cationized cellulose is produced by a production method that further comprises the following step (6):

step (6): a step of conducting a first drying treatment in which a product obtained in the step (5) is processed at a temperature of 50 to 120°C and a degree of vacuum of 13.4 to 53.3 kPa, and a second drying treatment in which the product following the first drying treatment is processed at a temperature of 90 to 150°C and a degree of vacuum of 13.3 kPa or less.

**12.** The skin detergent composition according to claim 10,
wherein the silane-modified cationized cellulose is produced by a production method that further comprises the following steps (7) and (8):

step (7): a step of adding an acid to a reaction product obtained in the step (5) for neutralization; and
step (8): a step of conducting a first drying treatment in which a product obtained in the step (7) is processed at a temperature of 50 to 120°C and a degree of vacuum of 13.4 to 53.3 kPa, and a second drying treatment in which the product following the first drying treatment is processed at a temperature of 90 to 150°C and a degree of vacuum of 13.3 kPa or less.

**13.** The skin detergent composition according to claim 10,
wherein the silane-modified cationized cellulose is produced by a production method that further comprises the following steps (7), (3) and (9):

step (7): a step of adding an acid to a reaction product obtained in the step (5) for neutralization;
step (3): a step of adding a water-compatible organic solvent or a mixed solvent composed of a water-compatible organic solvent and water so that, with respect to the cationized cellulose following neutralization, a water content in an entire solvent that comes into contact with the cationized cellulose becomes 10% by weight or less; and
step (9): a step of conducting a first drying treatment in which a product obtained in the step (3) is processed at a temperature of 50 to 120°C and a degree of vacuum of 13.4 to 53.3 kPa, and a second drying treatment in which the product following the first drying treatment is processed at a temperature of 90 to 150°C and a degree of vacuum of 13.3 kPa or less.

**INTERNATIONAL SEARCH REPORT**

International application No.
PCT/JP2009/006049

A. CLASSIFICATION OF SUBJECT MATTER
*A61K8/73*(2006.01)i, *A61K8/46*(2006.01)i, *A61Q5/02*(2006.01)i, *A61Q19/02*(2006.01)i

According to International Patent Classification (IPC) or to both national classification and IPC

B. FIELDS SEARCHED

Minimum documentation searched (classification system followed by classification symbols)
A61K8/00-8/99, A61Q1/00-99/00, C08B11/00-15/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CAplus/REGISTRY(STN), WPI

C. DOCUMENTS CONSIDERED TO BE RELEVANT

| Category* | Citation of document, with indication, where appropriate, of the relevant passages | Relevant to claim No. |
|---|---|---|
| X<br>Y | JP 2007-211167 A (Lion Corp.), 23 August 2007 (23.08.2007), entire text (Family: none) | 1-4,8-10<br>1-13 |
| Y | Shin Keshohin Gaku, Nanzando Co., Ltd., 18 January 2001 (18.01.2001), pages 440 to 445, 485 to 497, 3-1-1. Shampoo, 5 Body Care Keshohin, 5-1 Sekken, 5-2 Ekitai Body Senjoryo | 1-13 |
| P,X<br>P,Y | WO 2009/025354 A1 (Lion Corp.), 26 February 2009 (26.02.2009), entire text (Family: none) | 1-3,8,9<br>5-7,11-13 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>10 December, 2009 (10.12.09) | Date of mailing of the international search report<br>22 December, 2009 (22.12.09) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2009/006049

C (Continuation). DOCUMENTS CONSIDERED TO BE RELEVANT

| Category* | Citation of document, with indication, where appropriate, of the relevant passages | Relevant to claim No. |
|---|---|---|
| A | JP 2005-171089 A (Sumitomo Seika Chemicals Co., Ltd.), 30 June 2005 (30.06.2005), entire text (Family: none) | 1-13 |
| A | JP 2007-84680 A (Toho Chemical Industry Co., Ltd.), 05 April 2007 (05.04.2007), entire text (Family: none) | 1-13 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008289893 A
- JP SHO5538813 B
- JP 2000319139 A
- JP SHO512103 B
- JP SHO4735073 B
- JP HEI639481 B
- JP HEI8183801 B
- JP 2004155805 A
- JP SHO61195138 B
- JP 2007211167 A
- JP 2007216089 A
- JP HEI772201 B
- JP 2003300811 A


### Non-patent literature cited in the description


- Japanese Standards of Quasi-Drug Ingredients. Yakuji Nippo, Ltd, 2006